# EUROPEAN PATENT APPLICATION

(11) **EP 4 789 619 A1**
(43) Date of publication of application: **12.08.2026**
(21) Application number: 25156643.6
(22) Date of filing: 07.02.2025
(51) Int. Cl.: A61B 6/10

(54) **FOLDABLE RADIATION-PROTECTIVE SHIELD FOR OPERATING TABLE**

(71) Applicant: Texray AB, 412 92 Göteborg (SE)
(72) Inventor: APELL, Petra, 439 37 ONSALA (SE); GELLERSTEDT, Fredrik, 413 09 GÖTEBORG (SE)
(74) Representative: Öberg & Sjöberg AB

(57) **Abstract**

A foldable radiation-protective shield (20) for an operating table (2), comprising a holder (22) having an intended position attached to an operating table (2), a first arm (34) having a first end (32) attached to the holder (22), a second arm (38) having a first end (40) attached to the holder (22), the attachment of the first ends (32, 40) in the holder (22) is configured to allow at least one of the first and second arms (34, 38) to be pivoted from a first folded position substantially parallel to the other one to a second unfolded position, the shield (20) further comprising a flexible radiation-protective material (54) attached to the first and second arms (34, 38) and shaped to extend over at least part of the circle sector formed by the first and second arms (34, 38) in the second unfolded position. A system (90) and a use are also disclosed.

## Description

### Technical field

The technology proposed herein relates to a foldable radiation-protective shield for an operating table, and particularly to a foldable radiation-protective shield providing increased radiation protection in its unfolded position.

### Background

Ionizing radiation, such as X-ray, is commonly used in radiologically guided interventions, such as cardiology, or Interventional Radiology (IR). The clinical staff is exposed to the radiation during the procedures. Current standards and practices are based on the premise that any radiation dose may result in unfavorable health effects and can result in the development of radiation-induced diseases such as a specific type of cancer, glioblastoma. Reducing the radiation exposure of the clinical staff is therefore important.

The radiation can be direct or scattered. Radiation incident on a patient or a part of a patient will scatter, both at the point where the radiation impinges on the patient but also throughout the body of the patient. Further scattering occurs as the radiation impinges on the operating table.

During a radiologically guided intervention, or procedure, the patient is placed on an operating table. A source of radiation is provided above or underneath the operating table. The source of radiation may be positioned in various angular projections in relation to the patient. One or more radiation-protective drapes or coverings may be placed on the patient to prevent a major part of the radiation scattered in the body of the patient from irradiating out of the patient to reach the clinical staff.

Additionally, a radiation-protective curtain may be positioned along or under at least part of at least one long side of the operating table for preventing radiation emanating under the operation table, e.g. from radiation leakage from the source of radiation or from scattering, from reaching the clinical staff that typically stand along the left long side of the operating table for access to the interventional vascular entry points such as femoralis or radialis. Alternatively, the radiation-protective curtain may be placed higher up towards the head of the patient in such cases the clinical staff, for access to other interventional entry points such as subclavia or jugularis, chose to stand next to the patient's head.

A problem with conventional radiation-protective curtains positioned along or under a long side of the operating table is that certain procedures involve the source of radiation, which is typically carried at one end of a C-shaped arm with the detector carried at the other end, traveling under the operating table to a position at which it interferes with the radiation protective curtain. In other words, the source of radiation may travel to a position at which it emerges from under the operating table on the same side of the operating table that the clinical staff is positioned. If the source of radiation comes in contact with the curtain, a "collision" stop may be triggered which means that the C-shaped arm, or C arm, stops and must be reset, which interrupts the procedure and frustrates the clinical staff.

For that reason, it is not uncommon that sections of the operating table, in particular along the upper third or fourth of the table, e.g. in the area of the patient's head and torso, are devoid of a radiation-protective curtain in order to allow freedom of movement of the source of radiation. This of course decreases the protection for the clinical staff. In some cases, a separate smaller and/or shorter, radiation-protective curtain is used at such positions, which however affords less protection. In other cases, the curtain is designed as lamellaes to allow for flexibility and freedom of movement for the source of radiation.

In some cases, the patient is transported to the operating table from a transport bed (stretcher) by sliding the patient sideways over the gap between the beds. In other cases, the patient can step up onto the operating table. In both cases, close proximity and accessibility to the side of the bed is critical for patient safety.

### Objects of the proposed technology

The proposed technology aims at increasing the protection against radiation for the clinical staff while maintaining freedom of movement of the source of radiation.

It is a further object of the proposed technology to provide a foldable radiation-protective shield for an operating table.

It is yet a further object of the proposed technology to provide a foldable radiation-protective shield providing radiation protection in its unfolded position.

Another object of the proposed technology is to provide a foldable radiation-protective shield providing access for the patient to mount the operating table in its folded position without the need of dismantling the radiation-protective shield.

### Summary

A first aspect of the proposed technology thus concerns a foldable radiation-protective shield for an operating table, comprising:
- a holder having an intended position attached to an operating table, preferably to a side of an operating table,
- a first arm having a first end and a second end, the first end being attached to the holder,
- a second arm having a first end and a second end, the first end being attached to the holder,
   wherein the attachment of the first end of the first arm and the attachment of the first end of the second arm in the holder is configured to allow at least one of the first and second arm to be pivoted from a first folded position substantially parallel to the other one of the first and second arm, to a second unfolded position forming an angle to the other one of the first and second arm,
   wherein the foldable radiation-protective shield further comprises:
      - a flexible radiation-protective material attached to the first and second arms, the flexible radiation-protective material being shaped to extend over at least part of the circle sector formed by the first and second arms in the second unfolded position.

By the foldable radiation-protective shield according to the first aspect of the proposed technology it is possible to accommodate freedom of movement of the source of radiation, including when the source of radiation emerges from below the operating table at the side of the operating table the clinical staff is standing, while still maintaining protection from radiation directed upwards from under the operating table. In particular, when the one of the first and second arm carries a radiation-protective curtain as further described below, the pivoting of that arm to the unfolded position not only unfolds the flexible radiation-protective material extending at least over part of the circle sector formed by the first and second arms, but the pivoting further moves the radiation-protective curtain out of the way of the source of radiation, while protecting the clinical staff from radiation directed horizontally from the source of radiation under the operating table.

It is specified that the radiation-protective shield is foldable. Expressed differently, the radiation-protective shield is capable of folding from a compact folded state or shape to a more extensive or larger unfolded state or shape. The unfolding and folding in particular refers to the one of the first and second arm pivoting from a first folded position substantially parallel to the other one of the first and second arm, to a second unfolded position forming an angle to the other one of the first and second arm. The unfolding and folding of the radiation-protective shield may be performed manually by pivoting the arm, such as by pulling or pushing on the arm. Further, the unfolding of the radiation-protective shield may occur spontaneously as medical equipment, such as a source of radiation, contacts the radiation-protective shield under the operating table. Further, the folding of the radiation-protective shield may occur at least partially spontaneously due to the weight of the flexible radiation-protective material pulling the arm towards the other arm.

It is specified that the foldable shield is radiation-protective. Expressed differently, the covering protects against, or attenuates, ionizing radiation. Expressed differently, the shield may be an X-ray protective shield. The shield may comprise a radiation attenuating material, in particular an X-ray attenuating material. The shield may be arranged or configured to attenuate X-ray radiation with energies at least in the range 50-150 kV, such as 60-110 kV. The shield may be arranged or configured to attenuate X-ray radiation from a radiation source having a tube voltage of at least 40 kV, preferably at least 60 kV, and at the most 150 kV, preferably at the most 110 kV. For example, the covering may be arranged or configured to attenuate X-ray radiation from a radiation source having a tube voltage in the range of 40-150 kV, such as 60-110 kV. The covering may be arranged or configured to provide at least 0.05 mm, preferably at least 0.10 mm, more preferably at least 0.25 mm, most preferably at least 0.50 mm, and at the most 1.00 mm, preferably at the most 0.75 mm, more preferably at the most 0.50 mm Pb equivalence at any tube voltage in the range 60-110 kV. For example, the covering may be arranged or configured to provide 0.05 to 1.00 mm, preferably 0.10 to 1.00 mm, more preferably 0.25 to 1.00 mm, such as 0.25 to 0.75 mm or 0.30 to 0.60 mm, in particular at least 0.50 mm Pb equivalence, at any tube voltage in the range 60-110 kV. Pb equivalence is to be measured according to Inverse Broad Beam Geometry as described in IEC 61331-1:2014. The shield may be non-transparent, or non-translucent, to optical, or visible, light.

It is specified that the foldable radiation-protective shield is for an operating table. Expressed differently, the foldable radiation-protective shield is suitable for use with an operating table. In particular, the foldable radiation-protective shield is suitable for attachment to an operating table, preferably to the side of an operating table. The foldable radiation-protective shield may be rendered suitable for attachment to the operating table, preferably to the side of the operating table by providing an attachment structure on the holder, or by forming the holder to comprise an attachment structure, the attachment structure being configured for engaging the operating table, preferably the side of the operating table or for engaging a corresponding attachment structure provided on the operating table, preferably on or in the side of the operating table. The attachment structure on the holder may comprise any of hooks, fasteners (screws, rivets), magnets, or a weld. Preferably the attachment structure on the holder comprises a profile having a cross section engaging a corresponding cross section on a profile provided on or in the operating table or on or in the side of the operating table.

The holder may be elongated, such as an arm, beam or profile. Alternatively, the holder is a formed as a cuboid body, preferably a rectangular cuboid body.

It is specified that the holder has an intended position attached to an operating table, preferably to a side of an operating table. Expressed differently, the holder is configured to be attachable to the operating table, preferably to the side of an operating table, and, when so attached, has an intended position. Preferably the holder is only attachable to the operating table when placed in the intended position.

The side of an operating table encompasses a lateral vertical end surface of the operating table, as well as the upper and lower horizontal surfaces of the operating table surface and its underside, respectively, along the side of the operating table. In particular, the horizontal area extending between an edge of the surface of the operating table and a position within ¼ to 1/8, preferably within 1/16, of the width of the operating table from that edge may further be encompassed by the side of an operating table. Preferably the side of the operating table is limited to a lateral vertical end surface of the operating table. The side of the operating table may preferably be a long side of the operating table, i.e. a side of the operating table extending along the largest dimension of the operating table. The operating table may for example have a length of at least 1 m, preferably at least 1.5 m. The operating table may have a width of at least 0.4 m, preferably at least 0.6 m. The upper surface of the operating table may be provided at least 0.6 m, preferably at least 0.8 m above a floor surface upon which the operating table is placed. The operating table may be configured to provide selectable lifting or lowering of the operating table. An attachment structure may be provided on the operating table, preferably or in the side of the operating table for engaging an attachment structure provided on, or by, the holder.

Preferably the holder comprises an attachment structure engaging a corresponding attachment structure provided on the operating table, preferably on or in the side of the operating table, as described above. The attachment structure may comprise a profile such as rail with a corresponding attachment structure comprising a groove engaging and retaining the rail. The rail may for example be T-shaped, or L-shaped, in cross section and the groove may correspondingly be C-shaped, or J-shaped in cross section, or vice versa. Alternatively, the attachment structure may comprise discrete attachment points where fasteners such as screws or rivets attach the holder to the operating table or the side of the operating table. Additionally, the holder may be attached to the operating table or the side of the operating table by being integrally formed with the operating table or the side of the operating table, or by being welded or glued to the operating table or the side of the operating table. Additionally, an attachment structure comprised by the holder may comprise a clamping arrangement to clamp the side of the operating table or to clamp a structure such as a rail, profile, or sphere arranged on the operating table or in or at the side or the operating table. Additionally, an attachment structure comprised by the holder may comprise a plug or pin for being retained by a hole arranged on the operating table or in or at the side of the operating table.

An attachment structure associated with the holder may be formed integrally with the holder or may be attached to the holder.

The first arm may have a length of 10 to 100 cm, preferably 20 to 100 cm, such as 40 to 80 cm. The width of the first arm is preferably 1 to 5 cm, such as 1-3 cm. The height of the first arm is preferably 1 to 10 cm, such as 1 to 5 cm. The first arm may in particular be formed as an elongated profile or beam. The first arm may be made of metal, such as steel or aluminium, or made of plastic material, such as fiber reinforced plastic.

The first and second ends of the first arm may be considered opposite ends of the first arm. The first end may be considered an inner end or a proximal end, and the second end may be considered an outer end or a distal end.

It is specified that the first end of the first arm is attached to the holder. Expressed differently, the holder and the first end are connected mechanically to each other. In particular the first end is attached to the holder so that the holder supports the first arm in a substantially horizontal position when the holder is in the intended position. The first arm may be pivotably attached to the holder as further described below.

The second arm may have a length of 10 to 100 cm, preferably 20 to 100 cm, such as 40 to 80 cm. The width of the second arm is preferably 1 to 5 cm, such as 1-3 cm. The height of the second arm is preferably 1 to 10 cm, such as 1 to 5 cm. The second arm may in particular be formed as an elongated profile or beam. The second arm may be made of metal, such as steel or aluminium, or made of plastic material, such as fiber reinforced plastic.

Preferably the first and second arms have the same length, or alternatively, the difference in length of the first and second arms is not more than 20% of the length of the arm having the greatest length.

The first and second ends of the second arm may be considered opposite ends of the first arm. The first end may be considered an inner end or a proximal end, and the second end may be considered an outer end or a distal end.

The attachment of the first end or the first arm and the attachment of the first end of the second arm in the holder is configured to allow at least one of the first and second arm to be pivoted from a first folded position substantially parallel to the other one of the first and second arm, to a second unfolded position forming an angle to the other one of the first and second arm. Expressed differently, at least one of the first and second arm is pivotably attached to the holder via its first end. Preferably both the first and second arm is pivotably attached to the holder via its first end.

Expressed differently, the first end of the at least one of the first and second arm is attached to the holder so as be rotatable, or swivelable, in relation to the holder. The holder may for example comprise a pivot pin onto which the first end is placed. Further, the holder may comprise a hinge of which one leaf is attached to, or forms part of, the first end and the other leaf is attached to, or forms part of, the holder. In particular, the first end is pivotably attached to the holder so that the holder supports the arm in a substantially horizontal position when the holder is in the intended position, and so that the arm is pivotable, or rotatable, or swivelable, in a substantially horizontal plane when the holder is in the intended position.

Preferably the second arm is pivotably in relation to the first arm. Alternatively, both the first and the second arms are pivotable in relation to the holder. In any case, the first arm may generally be the arm closest to the operating table in the first folded position and with the holder in the intended position.

It is specified that the attachment of the first end of the first arm and the attachment of the first end of the second arm in the holder is configured to allow at least one of the first and second arm to be pivoted from a first folded position substantially parallel to the other one of the first and second arm, to a second unfolded position forming an angle to the other one of the first and second arm. Expressed differently, the first folded position comprises that the first and second arms are substantially parallel to each other, e.g. side by side in a horizontal plane, or one above the other in a vertical plane. The second unfolded position thus comprises that the first and second arm are non-parallel, i.e. not substantially parallel, but forms an angle, in particular an angle in a horizontal plane, between them. The angle is preferably 10 to 90°, such as 30° to 80°, for example 22.5° to 67.5°. Further expressed differently, the first folded position comprises that the distance between the second ends of the first and second arms is substantially the same as the distance between the first ends of the first and second arms, whereas the second unfolded position comprises that the distance second ends of the first and second arms is substantially larger, such as 2 to 10 times larger, than the distance between the first ends of the first and second arms.

The flexible radiation-protective material is capable of flexing, or deforming, when subjected to force, such as force applied on the flexible radiation-protective material. The flexible radiation-protective material is thus pliable. The flexible radiation-protective material may be at least partially elastic, or resilient, whereby the flexible radiation-protective material essentially, or substantially, resumes an initial shape and configuration once a force deforming it is no longer applied.

The flexible radiation-protective material may comprise, or consist of, a permeable fabric, or fibrous material. The permeable fabric may comprise filaments. The filaments may be arranged in a regular pattern. The filaments may extend in a uniform direction. Each filament may be of a solid composition that comprises a carrier substance and a radiopaque substance. For example, the solid composition may be a polymeric composite material. The filaments may be arranged in a regular pattern. It is understood that each filament may be a monofilament. It is further understood that the carrier substance and the radiopaque substance may be mixed, and that the radiopaque substance may be evenly distributed within the carrier substance. The carrier substance may comprise at least one polymer and the radiopaque substance may comprise at least one metal. For example, the polymer may be a thermoplastic polymer such as a polyvinyl chloride or a polyolefin and the metals may, as a powder, be as an element, oxide or alloy of for example Lead, Barium, Antimony, Bismuth, or Tungsten (Wolfram), and any combination thereof.

The fabric may be a woven fabric with the filaments forming the weft of the fabric. It is understood that the fabric has a warp, for example of polyester. More specifically, the first material may be any of the radiation-protective material described in WO2014163574A1. The fabric contributes to higher longevity of the foldable radiation-protective shield.

Alternatively, the flexible radiation-protective material may be, or comprise, an impermeable sheet. The impermeable sheet may be of a solid composition that comprises a carrier substance and a radiopaque substance. For example, the solid composition may be a polymeric composite material. It is further understood that the carrier substance and the radiopaque substance may be mixed, and that the radiopaque substance may be evenly distributed within the carrier substance. The carrier substance may comprise at least one polymer and the radiopaque substance may comprise at least one metal.

It is understood that the flexible radiation-protective material may be composed of several layers, for example two layers of fabric or two impermeable sheets. As specified above for the foldable radiation-protective shield, the flexible radiation-protective material may have at least 0.05, mm, preferably at least 0.10 mm, more preferably at least 0.25 mm, most preferably at least 0.50 mm, and at the most 1.00 mm, preferably at the most 0.75 mm, more preferably at the most 0.50 mm Pb equivalence at any tube voltage in the range 60-110 kV. For example, the covering may be arranged or configured to provide 0.05 to 1.00 mm, preferably 0.10 to 1.00 mm, more preferably 0.25 to 1.00 mm, such as 0.25 to 0.75 mm or 0.30 to 0.60 mm, most preferably at least 0.50 mm Pb equivalence at any tube voltage in the range 60-110 kV.

The flexible radiation-protective material may have a thickness of at least 0,5 mm, preferably at least 1 mm. The flexible radiation-protective material may have a thickness of at the most 10 mm, preferably at the most 5 mm, such as 0.5 to 10 mm, preferably 1 to 5 mm or 2 to 5 mm.

It is specified that the flexible radiation-protective material is attached to the first and second arms. Expressed differently, the first and second arms carry or support the flexible radiation-protective material. The flexible radiation-protective material may for example be attached to the first and second arms using any of adhesive or fasteners such as screws or rivets. As one example, the flexible radiation-protective material may be attached to the first and second arms by being sandwiched between parts, such as halves, of the first and second arms.

The flexible radiation-protective material is attached at at least one position to the first arm and at at least one position to the second arm. Preferably the flexible radiation-protective material is attached to at least 80%, such as at least 90% of the length of the first and second arms.

It is specified that the flexible radiation-protective material is shaped to extend over at least part of the circle sector formed by the first and second arms in the second unfolded position. Expressed differently, the flexible radiation-protective material has a shape corresponding to the circle sector, or the area encompassed by the first and second arms, in the second unfolded position.

The flexible radiation-protective material need not cover the full circle sector formed by the first and second arms, but it preferably covers a corresponding circle sector having a radius which is at least 80%, more preferably at least 90% of the length of the second arm. Expressed alternatively, the flexible radiation protective material preferably covers at least 50%, more preferably at least 70%, most preferably at least 80%, of the area of the circle sector formed by the first and second arms in the second unfolded position.

Further, the flexible radiation-protective material need not be shaped to have one side formed as the arc of the circle sector, i.e. a curved side extending between the first and second arms. Instead, the flexible radiation-protective material may be shaped to have a straight edge, whereby the flexible radiation-protective material is shaped substantially as a triangle with one side, edge or hypotenuse, extending along the second arm, a second side, edge, or adjacent extending along the first arm, and a third side, edge, or opposite extending from the first arm to the second arm and being opposite to the angle formed between the second and first arms.

The length of the sides of the flexible radiation-protective material along the first and second arms may preferably be at least 80%, more preferably at least 90%, up to 100% of the respective first and second arms.

In the first folded position the flexible radiation-protective material will generally fold along a fold line extending along the middle of the flexible radiation-protective material such that the flexible radiation-protective material hangs down between the first and second arms.

Preferably the flexible radiation-protective material covers the sides of the first and second arms that face away from each other in the first folded position.

This is advantageous in that it shields the first and second arms from the radiation, thus reducing scattering of radiation by the first and second arms.

The sides of the first and second arms that face away from each other in the first folded position may alternatively be referred to the outer sides of the first and second arms.

The flexible radiation-protective material may in particular cover the sides by being attached to the sides. The flexible radiation-protective material may be attached to the sides of the arms, or to any surface of the arms, by one or more of adhesive, fasteners passing through the flexible radiation-protective material, and by being sandwiched between each arm and a corresponding piece of material, such as a washer, plate or elongated strip of material fastened to the arm using a fastener, such as a screw or rivet, passing through the corresponding piece of material, the flexible radiation-protective material, and into the arm.

Preferably the flexible radiation-protective material is arranged and attached to the first arm so as to extend perpendicularly above the first arm when the holder is in the intended position.

As the first arm may generally be the arm closest to the operating table in the first folded position, this is advantageous in that it provides protection from radiation directed horizontally at the surface of the operating table or at the surface of a mattress positioned on the surface of the operating table. It further provides protection against radiation directed obliquely upwards from below the table into the gap between the first arm and the side of the operating table.

The part of the flexible radiation-protective material extending above the first arm may be considered a radiation-protective flap.

As the flexible radiation-protective material is flexible, the part of the material extending above the first arm can be bent by the patient or clinical staff if needed, e.g. by applying a force of 1-100 N to the upper edge of the material, and thus provides improved ergonomics to patient and clinical staff.

Preferably the flexible radiation-protective material extends at least 5 cm, more preferably at least 10 cm, most preferably at least 15 cm, above the first arm. Preferably flexible radiation-protective material extends 30 cm or less, more preferably 25 cm or less, most preferably 20 cm or less, above the first arm. Preferably the flexible radiation-protective material extends 5-30 cm, more preferably 5-25 cm, most preferably 10-20 cm, above the first arm.

It should however be noted that the flexible radiation-protective material may alternatively be arranged and attached to the first arm so as to not extend perpendicularly above the first arm, i.e. so as to end at or within the first arm, when the holder is in the intended position.

Preferably the flexible radiation-protective material is arranged and attached to the second arm so as to extend perpendicularly above the second arm when the holder is in the intended position.

This is also advantageous in that it provides protection from radiation directed horizontally at the surface of the operating table or at the surface of a mattress positioned on the surface of the operating table.

The part of the flexible radiation-protective material extending above the second arm may be considered a radiation-protective flap.

As the flexible radiation-protective material is flexible, the part of the material extending above the second arm can be bent by the patient or clinical staff if needed as described above, and thus provides improved ergonomics to patient and clinical staff.

Preferably the flexible radiation-protective material extends at least 5 cm, more preferably at least 10 cm, most preferably at least 15 cm, above the second arm. Preferably flexible radiation-protective material extends 30 cm or less, more preferably 25 cm or less, most preferably 20 cm or less, above the second arm. Preferably the flexible radiation-protective material extends 5-30 cm, more preferably 5-25 cm, most preferably 10-20 cm, above the second arm.

Preferably the flexible radiation-protective material is a permeable fabric that comprises filaments, each filament being of a solid composition that comprises a carrier substance and a radiopaque substance, and the fabric is a woven fabric with the filaments forming the weft of the fabric, or an impermeable sheet formed from a solid composition comprising a carrier substance and a radiopaque substance.

These are suitable flexible radiation-protective materials and have been described in further detail above.

When the filaments extend in a uniform direction, the uniform direction may be aligned with, or parallel with, the first and second arms in the first folded position. Worded differently, the uniform direction may be parallel to a horizontal direction along the side of the operating table when the holder is in the intended position. This increases the suppleness and longevity of the flexible radiation-protective material as the second arm is pivoted to and from the second unfolded position.

Preferably the flexible radiation-protective material comprises a permeable fabric that comprises filaments as described above. Such a permeable fabric has high tolerance, i.e. is more durable, to being bent, or deformed, when the flexible radiation-protective material is bent as the second arm is pivoted to and from the second unfolded position. This ensures longevity of the foldable radiation-protective shield.

Preferably the foldable radiation-protective shield further comprises:
- a radiation-protective flap comprising a radiation-protective material, attached to the first or second arm and configured to extend above the first or second arm, or upwards from the second arm, when the holder is in the intended position.

This provides radiation protection along and above the sides of the operating table. In particular, if the radiation-protective material is a flexible radiation-protective material as described further below, the radiation-protective flap can both extend above the side of the operating table to provide radiation protection when the patient is laying on the operating table, and bend to accommodate the patient when the patient is transferred, or lifted, e.g. in a supine or prone position, onto and off the operating table. There is thus no need to detach and reattach the radiation-protective flap from the first or second arm for these procedures. This improves ergonomics and efficiency of the handling of the patient.

The radiation-protective flap may extend from an upper edge, or upper horizontal edge, to a lower edge, or lower horizontal edge, the distance between the upper edge and the lower edge defining the height of the radiation-protective flap, i.e. the distance that the radiation-protective flap extends above the first or second arm in the intended position. The radiation-protective flap may further extend from a first edge, or first vertical edge, to a second edge, or second vertical edge, the distance between the first and second edges defining the length of the radiation-protective flap.

Preferably the flexible radiation-protective flap may have a height of at least 5 cm, more preferably at least 10 cm, most preferably at least 15 cm. Preferably the flexible radiation-protective flap may have a height of at the most 30 cm, more preferably at the most 25 cm, most preferably at the most 20 cm. Preferably the flexible radiation-protective flap has a height of 5-30 cm, more preferably 5-25 cm, most preferably 10-20 cm. The flexible radiation-protective flap may have a width, or length, of at 10 to 100 cm, preferably 20 to 100 cm, such as 40 to 80 cm, e.g. corresponding to the length of the first and second arm.

The flexible radiation-protective flap may have a thickness of at least 0,5 mm, preferably at least 1 mm. The flexible radiation-protective flap may have a thickness of at the most 10 mm, preferably at the most 5 mm.

Preferably the flexible radiation-protective flap is attached to the one of the first and second arm that is furthest from the operating table in the first folded position and with the holder in the intended position. Preferably the first arm is closest to the operating table in the first folded position and the radiation-protective flap is attached to the second arm.

Preferably the foldable radiation-protective shield further comprises:
- a radiation-protective curtain comprising a radiation-protective material attached to the first or second arm and configured to extend below the first or second arm, or downwards from the first or second arm, when the holder is in the intended position.

This is advantageous in that it further protects the clinical staff from radiation originating or scattered below the operating table. In addition, if being attached to the one of the first and second arm that pivots in relation to the holder in the second unfolded position, the radiation-protective curtain may be brought out of the way of the source of radiation below the operating table by pivoting the one arm towards the second unfolded position.

It is specified that the radiation-protective curtain is configured to extend below the first or second arm, i.e. the arm it is attached to, when the holder is in the intended position. Expressed differently, the flexible radiation-protective curtain extends vertically downwards from the first or second arm in the intended position.

The radiation-protective curtain may be attached to the first or second arm as described for the flexible radiation-protective material radiation-protective flap above.

The radiation-protective curtain may extend from an upper edge, or upper horizontal edge, to a lower edge, or lower horizontal edge, the distance between the upper edge and the lower edge defining the height of the radiation-protective curtain, i.e. the distance that the radiation-protective curtain extends below the first or second arm in the intended position. The radiation-protective curtain may further extend from a first edge, or first vertical edge, to a second edge, or second vertical edge, the distance between the first and second edges defining the length of the radiation-protective curtain.

Preferably the radiation-protective curtain has a height of at least 50 cm, more preferably at least 60 cm, most preferably at least 70 cm below the first or second arm.

The radiation-protective curtain may for example extend down to the floor on which the operating table stands, when the holder is in the intended position.

Preferably the first arm is closest to the operating table in the first folded position and the radiation-protective curtain is attached to the second arm which pivots in relation to the first arm and in relation to the holder.

Preferably the radiation-protective flap and the radiation-protective curtain are formed from the same piece of radiation-protective material.

Expressed differently, the same piece of radiation-protective material extends both above and below the first or second arm in the intended position. This is advantageous in that a single, or unbroken, piece of radiation-protective material making up both the radiation-protective flap and the radiation-protective curtain decreases the risk of radiation passing through the radiation-protective shield.

The height of the piece of radiation-protective material is thus at least the summed heights of the radiation-protective flap and the radiation-protective curtain.

The radiation-protective flap and the radiation-protective curtain are preferably attached to the same one of the first and second arm.

Preferably the foldable radiation-protective shield further comprises:
- a radiation-protective panel attached to the first or second arm and extending substantially parallel to the radiation-protective curtain,
whereby the attachment of the radiation-protective panel to the first or second arm is configured to allow the radiation-protective panel to move from a first lowered position, in which the radiation-protective panel overlies the radiation-protective curtain and/or the radiation-protective flap, to a second raised position in which the radiation-protective panel overlaps the radiation-protective curtain and/or the radiation-protective flap.

This is advantageous in that it provides further protection from radiation especially when the one the one of the first and second arm is in the second unfolded position at which the distance between the radiation-protective curtain, and the radiation-protective flap, if present, to the operating table is larger compared to with the arm in the first folded position. Specifically, by moving the radiation-protective panel to the second raised position, more of the scattered radiation can be stopped or at least attenuated.

It is specified that the radiation-protective panel can move from a first lowered position, in which the radiation-protective panel overlies the radiation-protective curtain and/or the radiation protective flap, to a second raised position in which the radiation-protective panel overlaps the radiation-protective curtain and/or the radiation-protective flap. Expressed differently, the first lowered position is one in which the radiation-protective panel is contained within the edges, or borders, of the radiation-protective curtain and/or flap. The second raised position is one in which the radiation-protective panel has been moved vertically upwards such that at least part of the radiation-protective panel extends beyond the upper edge, of the radiation-protective curtain and/or flap. Preferably, the second raised position is one in which at least part of the radiation-protective panel extends beyond the upper edge of the radiation-protective flap.

The attachment of the radiation-protective panel to the first or second arm may for example comprise a groove or rail attached to the first or second arm, and a corresponding rail or groove attached to the radiation-protective panel, the groove and rail being oriented vertically such that the radiation-protective panel may be raised to the second position by lifting, and thereby sliding, the rail or groove attached to the radiation-protective panel in relation to the groove or rail attached to the first or second arm. Regularly spaced holes or apertures may be provided in the groove or rail attached to the first or second arm for interaction with a latching pin associated with the rail or groove on the radiation-protective panel for maintaining the second raised position by preventing further movement of the rail vis-à-vis the groove.

The radiation-protective panel may comprise an inflexible radiation-protective material, or a flexible radiation-protective material as described above. Inflexible radiation-protective material may for example comprise lead sheets, plastic shields comprising radiation attenuating materials as described generally above, and radiation-protective glass shields, e.g. lead glass panes or sheets.

If the radiation-protective panel comprises flexible radiation-protective material, the radiation-protective panel may comprise an upper horizontal elongated support structure and a lower horizontal elongated support structure holding the flexible radiation-protective material and between which the flexible radiation-protective material extends. The support structures may in turn be attached to opposite ends of the vertical rail or groove attached to the radiation-protective panel.

Preferably the radiation-protective material of the radiation-protective flap and/or curtain is a flexible radiation-protective material being a permeable fabric that comprises filaments, each filament being of a solid composition that comprises a carrier substance and a radiopaque substance, and the fabric is a woven fabric with the filaments forming the weft of the fabric, or
an impermeable sheet formed from a solid composition comprising a carrier substance and a radiopaque substance.

These are suitable flexible radiation-protective materials, contribute and have been described in further detail above.

Additionally, when the filaments extend in a uniform direction, the uniform direction may be aligned with, or parallel with, the extension of the radiation-protective flap above the first or second arm, and/or the radiation-protective curtain, when the holder is in the intended position. Worded differently, the uniform direction may be parallel to the vertical direction when the holder is in the intended position. This increases the stiffness and longevity of the radiation-protective flap and curtain.

Preferably the flexible radiation-protective material comprises a permeable fabric that comprises filaments as described above. Such a permeable fabric has high tolerance, i.e. is more durable, to being bent, or deformed, when the radiation-protective flap is bent when a patient is transferred onto and off the operating table and/or when an object hits the curtain. This ensures longevity of the radiation-protective flap and curtain. Preferably the filaments of the permeable fabric extend vertically when the holder is in the intended position. This increases the stiffness of the radiation-protective flap thus maintaining it extending upwards above the first or second arm except when deflected, bent or deformed by a patient during transferral of the patient onto or off the operating table.

Preferably the attachment of both first ends of the first and second arms is configured to allow the first and second arms to pivot in relation to the holder and each other.

This is advantageous in that it allows the arm closest to the operating table with the holder in the intended position to be pivoted towards the side of the operating table so as to decrease the gap between the arm and the side of the operating table. This further reduces radiation from passing from below the operating obliquely upwards between the side of the operating table and the arm.

Additionally, in jugular or subclavian entry, this allows positioning the foldable radiation-protective shield at a head area of the patient whereby the first arm may be pivoted around the corner of the operating table to extend along the short side of the operating table and the second arm be allowed to not pivot.

The attachment of the first ends of the first and second arm may be configured to allow the arms to pivot in relation to the holder by comprising a pivot pin or hinge as described above.

Typically, the maximum extent of pivoting of the first arm is the same as the maximum extent of pivoting of the second arm. The maximum angle between the first and second arms is thus as described above up to 90°.

The pivoting of the arms is preferably in the same horizontal plane from the first folded position to the second unfolded position.

Preferably the attachment of at least one of the first end of the first arm and the first end of the second arm is configured to allow translation motion of the corresponding one of the first and second arm in relation to the holder, such as by the holder comprising a link having a first end pivotably attached in the holder and a second end, wherein the first end of the corresponding one of the first and second arm is pivotably attached to the second end of the link.

This allows further minimization of any gap between the arm closest to the operating table, such as typically the first arm, and the side of the operating table.

Typically, the extent of translation motion of the arm in relation to the holder is at the most 15 cm, preferably at the most 10 cm, such as 5 cm or less.
The link may have a length of 2 to 15 cm, such as 5 to 10 cm. the width and height of the link is preferably the same as the width and height of the arms. The link may be made of the same materials as the arms.

The pivotable attachment of the first end of the link to the holder, and of the first end of the corresponding one of the first and second arm to the second end of the link, may be provided by a pivot pin or hinge as described above.

The attachment of both the first end of the first arm and the second end of the second arm may configured to allow translation motion of the respective arm.

As an alternative, the link may be considered to form part of the first arm or second arm, however, in this alternative the flexible radiation-protective material is preferably not attached to the part of the arm made up by the link.

Preferably the holder comprises a rail configured to slidingly enter into, and be retained by, a groove provided along the side of an operating table, for attaching the holder to the side of the operating table, or to the operating table.

The rail is on example of an attachment structure provided on or in or being associated with the holder.

This provides a secure attachment of the holder to the operating table. The groove and rail may provide additional protection from radiation originating from below the operating table. The groove may for example be C shaped and the rail T-shaped. The inverse arrangement, with the holder comprising the grove and the side of an operating table comprising the rail, is also possible.

The rail may be formed integrally with the holder or may be a separate elongated rail extending from the holder. In the latter case, the separate elongated rail may be provided with a radiation-protective flap, and optionally also a radiation-protective curtain, as described above. The rail may be made from the same materials as the holder and as the first arm.

Alternatively, a separate flexible radiation-protective shield, comprising a support structure such as an elongated arm, beam or profile having an intended position attached to the side of an operating table, and further comprising a radiation-protective flap, and optionally also a radiation-protective curtain, made from the same piece of flexible radiation-protective material as described above, attached to the support structure, may be used together with the foldable radiation-protective shield. Such a separate flexible radiation-protective shield may advantageously be attached to the side of the operating table adjacent to the foldable radiation-protective shield so that one vertical edge of the flexible radiation-protective material of the separate flexible radiation-protective shield overlaps one vertical edge of the flexible radiation-protective material of the foldable radiation-protective shield.

A second aspect of the proposed technology concerns a system comprising an operating table and a foldable radiation-protective shield according to the first aspect of the proposed technology.

The operating table and the foldable radiation-protective shield are as described above.

A third aspect of the proposed technology concerns the use se of the foldable radiation-protective shield according the first aspect of the proposed technology, or the system according to the second aspect of the proposed technology, for protecting clinical staff from radiation during a medical intervention comprising the use of radiation.

The medical intervention may comprise a radiologically guided intervention, such as cardiology, or Interventional Radiology (IR). The medical intervention, or medical procedure, may comprise diagnostic and/or surgical procedures. The medical intervention may be a fluoroscopic, or fluoroscopy, assisted intervention.

The use may comprise pivoting one of the first and second arm from the first folded position to the second unfolded position.

The use may further comprise moving the radiation-protective panel from the first lowered position to the second raised position.

The clinical staff may comprise any personnel present during the medical intervention.

The third aspect of the proposed technology may alternatively be considered a method of protecting clinical staff from radiation during a medical intervention comprising the use of radiation, comprising the steps of:
- providing:
   - the radiation-protective shield according to the first aspect of the proposed technology and an operating table, or
   - the system according to the second aspect of the proposed technology, and
- attaching the radiation-protective shield to the operating table, preferably to the side of the operating table.

### Brief description of the drawings

A more complete understanding of the abovementioned and other features and advantages of the technology proposed herein will be apparent from the following detailed description of preferred embodiments in conjunction with the appended drawings, wherein:

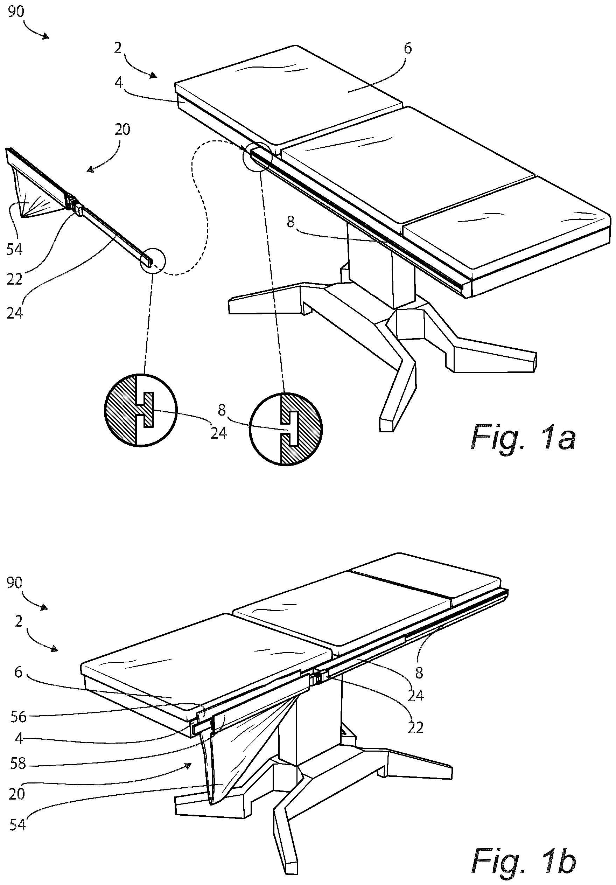

Fig. **1a** shows a perspective view of a first embodiment of a foldable radiation-protective shield according to the first aspect of the proposed technology together with an operating table.
Fig. 1b shows a perspective view of the first embodiment of the foldable radiation-protective shield shown in Fig. 1a attached to the side of the operating table.

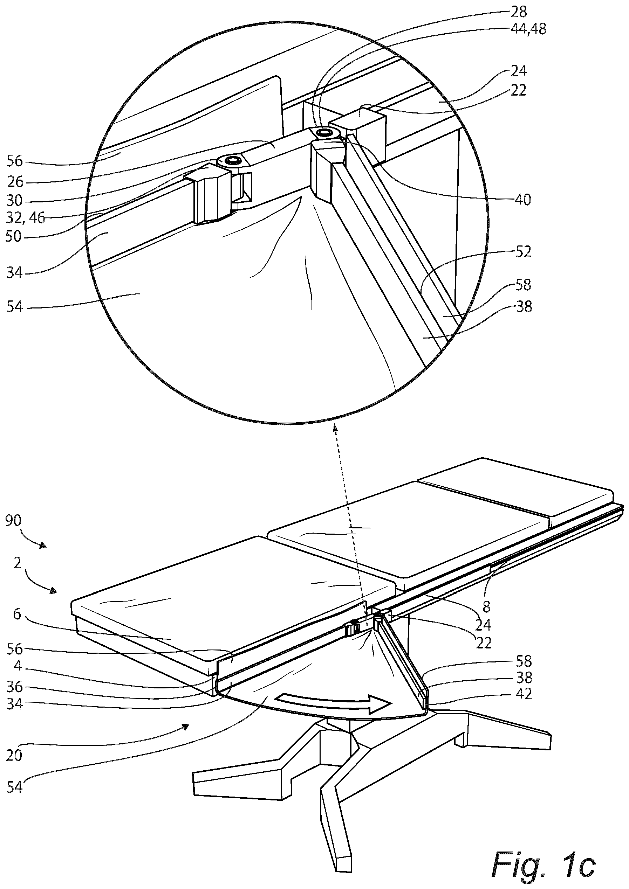

**Fig. 1c** shows a perspective view of the first embodiment of the foldable radiation-protective shield shown in Fig. 1b with the second arm in the second unfolded position.

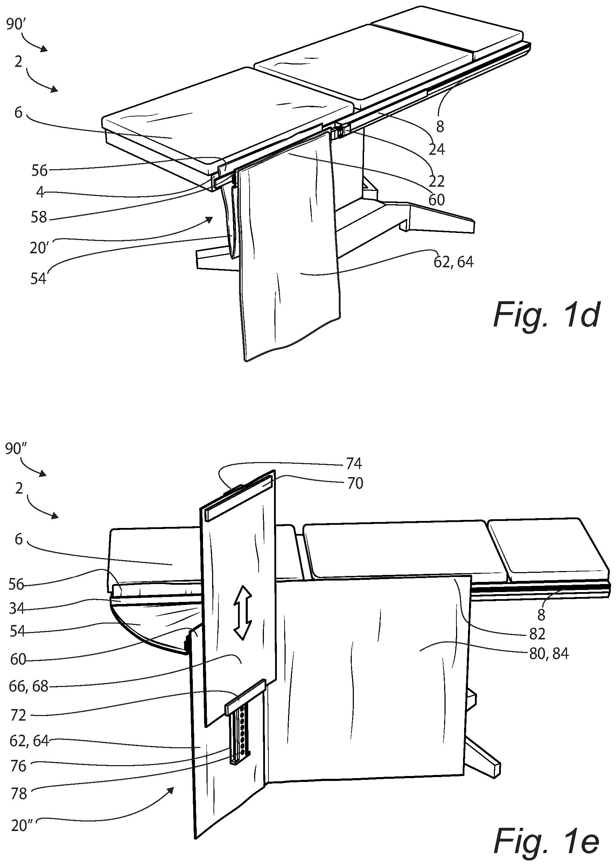

**Fig. 1d** shows a perspective view of a second embodiment of a foldable radiation-protective shield according to the first aspect of the proposed technology attached to the side of an operating table, the second embodiment further comprising a radiation-protective flap and a radiation-protective curtain attached to the second arm.
**Fig. 1e** shows a perspective view of a third embodiment of a foldable radiation-protective shield according to the first aspect of the proposed technology attached to the side of an operating
table, the third embodiment being shown with the second arm in the second unfolded position and further comprising a radiation protective curtain and a radiation-protective panel in the second raised position, as well as comprising a radiation-protective curtain and radiation-protective flap attached to the rail attaching the holder to the side of the operating table.

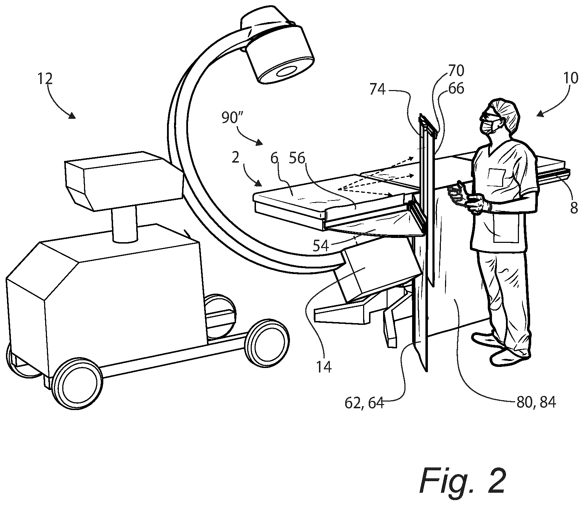

**Fig. 2** shows a perspective view of the third embodiment of the foldable radiation-protective shield together with a C-arm imaging device and a clinical staff member.

### Detailed description

In the figures and the below description, the same reference numerals are used for the same or related features. One or more ' added to a reference number indicates that the feature so designated is a variant of the feature bearing the reference number without the '.

**Fig 1a** shows an operating table 2 having a side 4 and carrying a mattress 6 onto which a patient (not shown) may be placed during a clinical procedure. The side 4 of the operating table 2 is provided with an elongated groove 8 representing an attachment structure, e.g. for attaching medical equipment to the side 4 of the operating table 2, or, as in this case, attaching the foldable radiation-protective shield 20. Foldable radiation-protective shield 20 comprises, as is best seen in Fig. 1c, a holder 22 formed as a cuboid body. An attachment structure, shown as an elongated rail 24, is attached to one side of the holder 22 and has a cross-sectional profile that matches the cross-sectional profile of the elongated groove 8 provided on the side 4 of the operating table 2, as best seen in the enlargements in Fig. 1a.

This allows the holder 22 to be attached to the side 4 of the operating table 2 and thus allows the holder to assume its intended position attached to the operating table 2, such as to the side 4 of the operating table 2, as shown in **Fig. 1b****.**

Although the elongated groove 8 is shows as extending along only a part of the side 4 of the operating table 2, the elongated groove may alternatively extend along all of the side 4 of the operating table 2. Further, although the embodiments shown in the figures use an attachment structure in the form of elongated rail 24 associated with the holder 22 and an elongated groove 8 associated with the side 4 of the operating table, the reverse arrangement, as well as arrangements where the holder 22 is attached to operating table 2 directly using fasteners such as screws or rivets, is equally possible.

Both fig. 1a and 1b show the foldable radiation-protective shield 20 in its folded configuration.

The holder 22 further comprises a link 26 having a first end 28 pivotably attached in the holder and a second end 30 to which a first end 32 of a first arm 34 is pivotably attached. This allows both translational and pivoting movement of the first arm 34 and thus allows the first arm 34, including its first end 32 and its second end 36, to be brought close the side 4 of the operating table 2 to prevent radiation from passing upwards between the side 4 of the operating table 2 and the first arm 34. A second arm 38 having a first end 40 and a second end 42 is also pivotably attached to the holder 22 via its first end 40, however in this case without a link 26 since the second arm 38 generally does not need to be capable of translational movement. However, the second arm 38 may also be implemented as a copy of the first arm 34 and link 26. As seen, the first arm 34 and the second arm 38 may generally be implemented as metal, e.g. aluminium, profiles. The length of the second arm 38 is 70 cm, the length of the first arm 34 is 60 cm, and the length of the linker 26 is 10 cm. The width and height of the first and second arms 34, 38 are 1 cm and 4 cm, respectively. The width and height of the linker 26 is 2 cm and 4 cm, respectively. The holder 22 and the link 26 may as shown be formed of solid metal, e.g. aluminium. The pivotable attachments between the first ends 32, 40 of the first and second arms 34, 38, and the second end 30 of the link 26 and the holder 22, respectively, as well as the pivotable attachment of the first end 28 of the link 26 in the holder 22, may as seen be provided using pivot pins 44. The first ends 32, 40 of the first and second arms 34, 38 may as shown have a larger width and height, and my as shown be implemented as one leaf or part 46, 48 of a hinge further comprising the pivot pin 44 and the other part of the respective hinge provided by the second end 30 of the link 26 and the holder 22, respectively.

To the outer sides 50, 52 of the first and second arms 34, 38 is attached a flexible radiation-protective material 54 shaped to extend over at least part of the circle sector formed by the first and second arms 34, 38 when, in this case, the second arm 38 is in the second unfolded position as shown in Fig. 1c. The flexible radiation-protective material 54 is attached to the first and second arms 34, 38 using adhesive or a plurality of fasteners such as screws and washers, not shown.

The flexible radiation-protective material 54 is generally shaped as a circle sector having a radius corresponding to the length of the second arm 38. In addition, as shown, the flexible radiation-protective material 54 is further shaped to extend above the first arm 34 and above the second arm 38 to form first and second radiation-protective flaps 56, 58 having heights of 10 and 5 cm, respectively. This further reduces radiation from passing from below the operating table 2 between the side 4 of the operating table 2 and the first arm 34. Further, by having the flexible radiation-protective material 54 attached to the outer sides 50, 52 of the first and second arms 34, 38, the risk of the first and second arms 34, 38 scattering radiation from below the operating table 2 decreases.

The flexible radiation-protective material 54 is a permeable fabric that comprises monofilaments arranged in a regular pattern and extending in a uniform direction, for example as described in WO2014163574A1. Each filament is solid composition that comprises a carrier substance of polyolefin and radiopaque substance of Antimony and Bismuth powder that are uniformly mixed. The fabric is a woven fabric with the filaments forming the weft of the cloth and with a warp of polyester. The X flexible radiation-protective material 54 can attenuate X-ray radiation from a radiation source having a tube voltage of between 40 to 150 kV. The flexible radiation-protective material used in the embodiments of the foldable radiation-protective shield shown in the figures has at least 0.50 mm Pb equivalence at any tube voltage in the range 60-110 kV. The filaments of the flexible radiation-protective material 54 are oriented along the first arm 34 so as to ease the folding and unfolding of the flexible radiation-protective material 54 as the second arm 38 is pivoted to and from the unfolded second position shown in **Fig. 1c****,** and so as to ease the forming of a fold necessary for the flexible radiation-protective material 54 to hang down between the first and second arms 34, 38 as shown in Fig. 1a and Fig. 1b.

The operating table 2 and the foldable radiation-protective shield 20 constitute a system 90.

**Fig. 1d** shows a perspective view of a second embodiment 20' of a foldable radiation-protective shield according to the first aspect of the proposed technology attached to the side 4 of an operating table 2. The second embodiment further comprises a radiation-protective flap 60 and a radiation-protective curtain 62 attached to the second arm 38. Specifically, the radiation-protective flap 60 and a radiation-protective curtain 62 are formed from the same, i.e. a single, piece of flexible radiation-protective material 64. The flexible radiation-protective material 64 is of the same type as the flexible radiation-protective material 54 but has the filaments arranged in the vertical direction so as to increase stiffness of the radiation-protective flap 60 and a radiation-protective curtain 62. AS seen, the radiation-protective flap 60, having a height of 5 cm, provides protection against radiation scattered horizontally from a patient on the mattress 6 on the operating table 2, whereas the radiation-protective curtain 62, having a height of 70 cm and extending from the second arm 38 down to the floor on which the operating table 2 is positioned, provides protection against radiation originating from below the operating table 2.

Advantageously, as seen in Fig. 1d and as further seen in **Fig. 2****,** by providing the radiation-protective flap 60 and a radiation-protective curtain 62 attached to the second arm 38, the pivoting of the second arm 38 simultaneously moves the radiation-protective curtain 62 out of the way of e.g. a source of radiation moving out from under the operating table, while the flexible radiation-protective material 54 extends to cover the circle sector formed by the first and second arms 34, 38. Accordingly, even when the second arm 38 has to be pivoted to the second unfolded position so as to provide space for the source of radiation, the flexible radiation-protective material 54 and the radiation-protective curtain 62 protects clinical staff 10 from the radiation, as further illustrated in Fig. 2.

The operating table 2 and the foldable radiation-protective shield 20' together constitute a system 90'.

In order to provide further protection against radiation originating or being scattered below the operating table 2, separate radiation-protective curtains may be attached to the remainder of the side 4 of the operating table 2. Advantegously, the foldable radiation-protective shield 20' further comprises, or is used with, such a further radiation-protective curtain and radiation-protective flap as shown in Fig. 1e.

**Fig. 1e** thus shows a perspective view of a third embodiment 20'' of a foldable radiation-protective shield according to the first aspect of the proposed technology attached to the side 4 of an operating table 2, the third embodiment being shown with the second arm 38 in the second unfolded position and further comprising a radiation protective curtain 62, radiation-protective flap 60, and a radiation-protective panel 66 in the second raised position. The radiation-protective panel 66 comprises a flexible radiation-protective material 68 being of the same type as the flexible radiation-protective material 64 and being provided between an upper horizontal elongated support structure 70 and a lower horizontal elongated support structure 72. These support structures 70, 72 are in turn attached to opposite ends of a profile with an elongated vertical groove 74 which slidingly engages an elongated vertical rail 76 attached to the second arm 38 and extending on the outside of radiation-protective curtain 62. Regularly spaced holes or apertures 78 may be provided in the elongated vertical rail 76 attached to the second arm 38 for interaction with a latching pin (not shown) associated with the elongated vertical groove 74 on the radiation-protective panel 66 for arresting the radiation-protective panel 66 at a second raised position, as shown in Fig. 1e, vis-à-vis a first lowered position (not shown) in which the radiation-protective panel 66 overlies the radiation-protective curtain 62 and radiation protective flap 60.

The radiation-protective panel 66 provides further protection from scattered radiation from a patient placed on the mattress 6 when the second arm 38 is in the second unfolded position. This is because, in the second unfolded position, the distance between the patient and the radiation-protective flap 60 is larger than in the first folded position, accordingly, the protection provided by the radiation-protective flap 60 decreases. In other words, with a larger distance between the source of the scattered radiation, i.e. the patient, and the radiation-protective flap 60, more of the scattered radiation will pass above the upper edge of the radiation-protective flap 60 to irradiate clinical staff 10 positioned at the side 4 of the operating table. By raising the radiation-protective panel 66 to its second raised position when the second arm 38 is in the second unfolded position, more of this scattered radiation is blocked by the radiation-protective panel 66 as it extends above the radiation-protective flap 60.

The foldable radiation-protective shield 20" further comprises a further radiation-protective curtain 80 and further radiation-protective flap 82 attached to the elongated rail 24 attaching the holder 22 to the side 4 of the operating table 2. This advantageously provides even further protection from radiation originating below the operating table 2 and scattered horizontally by a patient on the mattress 6. The further radiation-protective flap 82 and the further radiation-protective curtain 80 are formed from the same, i.e. a single, piece of flexible radiation-protective material 84. The flexible radiation-protective material 84 is of the same type as the flexible radiation-protective material 64. As seen, the radiation-protective flap 82, having a height of 5 cm above the elongated rail 24, provides protection against radiation scattered horizontally from a patient on the mattress 6 on the operating table 2, whereas the radiation-protective curtain 80, having a height of 70 cm and extending from the elongated rail 24 down to the floor on which the operating table 2 is position, provides protection against radiation originating from below the operating table 2. The width of the further radiation-protective curtain 80 and further radiation-protective flap 80 is here 1 m.

The operating table 2 and the foldable radiation-protective shield 20" together constitute a system 90".

**Fig. 2** shows a perspective view of the third embodiment 20'' of the foldable radiation-protective shield together with a C-arm imaging device 12 and a clinical staff member 10. As seen, by pivoting the second arm 38 to the second unfolded position, the radiation source 14 of the C-arm imaging device 12 can travel below the operating table 2 to a position at which it would otherwise contact the radiation-protective curtain 62: this is prevented by pivoting the second arm 38 while the flexible radiation-protective material 54 protect against radiation from the radiation source 14 travelling upwards towards the circle sector formed by the first and second arms 34, 38. The radiation protective panel 66, by being moved to the second raised position, provides further protection from radiation scattered by the patent as indicated by the dashed arrows. Thus, the clinical staff member 10 is protected from radiation by the flexible radiation-protective material 54, the radiation protective curtain 62 and the radiation protective flaps 60 and 56, the radiation protective panel 66, and the further radiation-protective curtain 80 and further radiation-protective flap 82.

Although in the above description of the figures it is generally the second arm 38 that pivots to the second unfolded position, the opposite movement, i.e. with the first arm pivoting away from the second arm, is also possible. Specifically, if considering e.g. Fig. 1d, it can be seen that for medical interventions to the head of the patient, the holder 22 may be moved further towards the head part of the operating table 2, i.e. towards the left end of the side 4 of the operating table 2. Once the holder 22 has been positioned at the corner of the operating table 2, the linker 26 and first arm 34 may be pivoted 90° away from the second arm 38 so as to extend along the short side of the operating table 2. In such a position, the second arm 38 is typically allowed to continue to extend substantially parallel to the long side, i.e. side 4, of the operating table, or be pivoted only partly towards the short side of the operating table 2, to provide the angle and circle section between the first and second arms 34, 38 over which the flexible radiation-protective material 54 extends at least partly.

As is further evident from Fig. 2 a patient is able to easily mount and dismount, i.e. get up on, or get down from, the operating table 2 when the second arm 38 is in the first folded position and the radiation protective panel 66 is in the first lowered position. There is thus no need of dismantling or removing the foldable radiation-protective shield 20 when the patient mounts or dismounts the operating table 2, or is lifted onto or off the operating table 2.

### Item list

- 2: operating table
- 4: side of operating table
- 6: mattress
- 8: elongated groove
- 10: clinical staff
- 12: c-arm imaging device
- 14: radiation source
- 20, 20', 20": foldable radiation-protective shield
- 22: holder
- 24: elongated rail
- 26: link
- 28: first end
- 30: second end
- 32: first end
- 34: first arm
- 36: second end
- 38: second arm
- 40: first end
- 42: second end
- 44: pivot pin
- 46: part of hinge
- 48: part of hinge
- 50: outer side of first arm
- 52: outer side of second arm
- 54: flexible radiation-protective material
- 56: radiation-protective flap
- 58: radiation-protective flap
- 60: radiation-protective flap
- 62: radiation-protective curtain
- 64: flexible radiation-protective material
- 66: radiation-protective panel
- 68: flexible radiation-protective material
- 70: upper horizontal elongated support structure
- 72: lower horizontal elongated support structure
- 74: elongated vertical groove
- 76: elongated vertical rail
- 78: holes
- 80: further radiation-protective curtain
- 82: further radiation-protective flap
- 84: flexible radiation-protective material
- 90, 90', 90": system

## Claims

1. A foldable radiation-protective shield (20) for an operating table (2), comprising:
- a holder (22) having an intended position attached to an operating table (2), preferably to a side (4) of an operating table (2),
- a first arm (34) having a first end (32) and a second end (36), the first end (32) being attached to the holder (22),
- a second arm (38) having a first end (40) and a second end (42), the first end (40) being attached to the holder (22),
wherein the attachment of the first end (32) of the first arm (34) and the attachment of the first end (40) of the second arm (38) in the holder (22) is configured to allow at least one of the first and second arm (34, 38) to be pivoted from a first folded position substantially parallel to the other one of the first and second arm (34, 38), to a second unfolded position forming an angle to the other one of the first and second arm (34, 38),
wherein the foldable radiation-protective shield (20) further comprises:
- a flexible radiation-protective material (54) attached to the first and second arms (34, 38), the flexible radiation-protective material (54) being shaped to extend over at least part of the circle sector formed by the first and second arms (34, 38) in the second unfolded position.

2. The foldable radiation-protective shield (20) according to claim 1, wherein the flexible radiation-protective material covers the sides (50, 52) of the first and second arms (34, 38) that face away from each other in the first folded position.

3. The foldable radiation-protective shield (20) according to any preceding claim, wherein the flexible radiation-protective material (54) is arranged and attached to the first arm (34) so as to extend perpendicularly above the first arm (34) when the holder (22) is in the intended position.

4. The foldable radiation-protective shield (20) according to any preceding claim, wherein the flexible radiation-protective material (54) is arranged and attached to the second arm (38) so as to extend perpendicularly above the second arm (38) when the holder (22) is in the intended position.

5. The foldable radiation-protective shield (20) according to any preceding claim, wherein the flexible radiation-protective material (54) is a permeable fabric that comprises filaments, each filament being of a solid composition that comprises a carrier substance and a radiopaque substance, and the fabric is a woven fabric with the filaments forming the weft of the fabric, or
an impermeable sheet formed from a solid composition comprising a carrier substance and a radiopaque substance.

6. The foldable radiation-protective shield (20) according to any preceding claim, further comprising:
- a radiation-protective flap (60) comprising a radiation-protective material (64), attached to the first or second arm (34, 38) and configured to extend above the first or second arm (34, 38) when the holder (22) is in the intended position.

7. The foldable radiation-protective shield (20) according to any preceding claim, further comprising:
- a radiation-protective curtain (62) comprising a radiation-protective material (64) attached to the first or second arm (34, 38) and configured to extend below the first or second arm (34, 38) when the holder (22) is in the intended position.

8. The foldable radiation-protective shield (20) according to claim 7, wherein the radiation-protective flap (60) and the radiation-protective curtain (62) are formed from the same piece of radiation-protective material (64).

9. The foldable radiation-protective shield (20) according to any of the claims 6-8, further comprising:
- a radiation-protective panel (66) attached to the first or second arm (34, 38) and extending substantially parallel to the radiation-protective curtain (62),
whereby the attachment of the radiation-protective panel (66) to the first or second arm (38) is configured to allow the radiation-protective panel (66) to move from a first lowered position, in which the radiation-protective panel (66) overlies the radiation-protective curtain (62) and/or the radiation-protective flap (60), to a second raised position in which the radiation-protective panel (66) overlaps the radiation-protective curtain (62) and/or the radiation-protective flap (60).

10. The foldable radiation-protective shield (20) according to any of the claims 6-9, wherein the radiation-protective material (64) is a flexible radiation-protective material being a permeable fabric that comprises filaments, each filament being of a solid composition that comprises a carrier substance and a radiopaque substance, and the fabric is a woven fabric with the filaments forming the weft of the fabric, or
an impermeable sheet formed from a solid composition comprising a carrier substance and a radiopaque substance.

11. The foldable radiation-protective shield (20) according to any preceding claim, wherein the attachment of both first ends (32) of the first and second arms (34, 38) is configured to allow both the first arm (34) and the second arm (3) to pivot in relation to the holder (22) and each other.

12. The foldable radiation-protective shield (20) according to any preceding claim, wherein the attachment of at least one of the first end (32) of the first arm (34) and the first end (40) of the second arm (38) is configured to allow translation motion of the corresponding one of the first and second arm (34, 38) in relation to the holder (22), such as by the holder (22) comprising a link (26) having a first end (28) pivotably attached in the holder (22) and a second end (30), wherein the first end (32, 40) of the corresponding one of the corresponding one of the first and second arm (34, 38) is pivotably attached to the second end (30) of the link (26).

13. The foldable radiation-protective shield 820) according to any preceding claim, wherein the holder (22) comprises a rail (24) configured to slidingly enter into, and be retained by, a groove (8) provided along the side (4) of an operating table (2), for attaching the holder (22) to the side (4) of the operating table (2) .

14. A system (90) comprising an operating table (2) and a foldable radiation-protective shield (20) according to any of the preceding claims.

15. Use of the foldable radiation-protective shield (20) according to any of the claims 1-13, or the system (90) according to claim 14, for protecting clinical staff (10) from radiation during a medical intervention comprising the use of radiation.
